# EUROPÄISCHE PATENTSCHRIFT

(11) **EP 2 037 988 B1**
(45) Veröffentlichungstag und Bekanntmachung des Hinweises auf die Patenterteilung: **26.09.2012**
(21) Anmeldenummer: 07718370.5
(22) Anmeldetag: 04.04.2007
(51) Int. Cl.: A61M 5/32

(54) **INJEKTIONSSPRITZE**
INJECTION SYRINGE
SERINGUE

(30) Priorität: 06.04.2006 AT 5892006
(43) Veröffentlichungstag der Anmeldung: 25.03.2009
(73) Patentinhaber: Pharma Consult Ges.m.b.H. & Co Nfg KG, A-1210 Wien (AT)
(72) Erfinder: PICKHARD, Ewald, A-2203 Grossebersdorf (AT)
(74) Vertreter: Burger, Hannes
(86) Internationale Anmeldenummer: PCT/AT2007/000153
(87) Internationale Veröffentlichungsnummer: WO 2007/112470

(56) Entgegenhaltungen:
- EP-A- 1 514 566
- WO-A-91/08788
- DE-U1- 29 821 609
- US-A- 5 256 151
- US-A- 5 263 934
- US-A1- 2005 277 880
- US-B1- 6 613 016

## Beschreibung

Die Erfindung betrifft eine Injektionsspritze für den Einmalgebrauch, mit einem Spritzenzylinder, einem durch eine Kolbenstange im Spritzenzylinder verschiebbaren Kolbenstopfen, einer im Spritzenzylinder aufgenommene Nadelbaugruppe, wobei diese einen eine Injektionsnadel aus nicht rostendem Stahl auf einem Teil ihrer Länge umschließenden Nadelhalter aus Kunststoff sowie einen Dichteinsatz umfasst, und mit Kupplungsmitteln zum Verbinden der Kolbenstange mit der Nadelbaugruppe, um nach erfolgter Injektion die Nadelbaugruppe in den Spritzenzylinder zurück zu ziehen, und der Spritzenzylinder, die Nadelbaugruppe sowie der Kolbenstopfen einen Innenraum zur Aufnahme einer Injektionslösung bildern.

Die US 2005/0277880 A1 offenbart eine Injektionsspritze für den Einmalgebrauch, die aus einem Spritzenzylinder, einem durch eine Kolbenstange im Spritzenzylinder verschiebbaren Kolbenstopfen und einer im Spritzenzylinder aufgenommenen Nadelbaugruppe besteht. Die Nadelbaugruppe umfasst lediglich einen Nadelhalter, in dem die Injektionsnadel feststehend gehaltert ist. Zwischen dem Nadelhalter der Nadelbaugruppe und der Kolbenstange sind Kupplungsmittel zum Verbinden der Kolbenstange mit der Nadelbaugruppe vorgesehen, um nach erfolgter Injektion die Nadelbaugruppe in den Spritzenzylinder zurückziehen zu können. Der Innenraum zur Aufnahme der Injektionslösung wird durch den Spritzenzylinder, den Nadelhalter der Nadelbaugruppe sowie dem Kolbenstopfen gebildet. Zusätzlich kann ein Dichtungsring am Umfang des Nadelhalters zur dichtenden Anlage an der Innenwand des Spritzenzylinders vorgesehen sein. Dieser Spritzentyp dient zum kurzfristigen Aufnehmen und Abgeben von Injektionslösungen.

Die DE 298 21 609 U1 beschreibt ebenfalls eine Injektionsspritze für den Einmalgebrauch mit einem Spritzenzylinder, einem durch eine Kolbenstange im Spritzenzylinder verschiebbaren Kolbenstopfen sowie eine im Spritzenzylinder aufgenommene Nadelbaugruppe. Die Nadelbaugruppe umfasst einen Nadelhalter aus Kunststoff, in dem eine Injektionsnadel feststehend gehaltert ist. Der Nadelhalter ist über Rastmittel im Spritzenzylinder für die Aufnahme und Abgabe der Injektionslösung gehaltert. Weiters sind Kupplungsmittel zwischen dem Kolbenstopfen sowie der Nadelbaugruppe vorgesehen, um nach erfolgter Injektion die Nadelbaugruppe in den Spritzenzylinder zurückziehen zu können. Für die kurzfristige Aufnahme der Injektionslösung ist der Innenraum durch den Spritzenzylinder, den Nadelhalter sowie dem davon distanzierten Kolbenstopfen gebildet. Auch diese Injektionsspritze dient nur zur kurzfristigen Aufnahme und Abgabe von Injektionslösungen.

Aus der US 5,256,151 A ist eine Injektionsspritze für den Einmalgebrauch bekannt geworden, die einen Spritzenzylinder, einen durch eine Kolbenstange im Spritzenzylinder verschiebbaren Kolbenstopfen sowie eine im Spritzenzylinder aufgenommene Nadelbaugruppe umfasst. Die Nadelbaugruppe ihrerseits umfasst einen eigenen Nadelträger mit einer an diesem fix angeordneten Injektionsnadel. Weiters ist im vorderen Abschnitt der Injektionsspritze ein. ein zusätzliches Zwischenstück bzw. zusätzlicher Nadelhalter angeordnet, der über eine Gewindeverbindung mit dem Spritzenzylinder verbunden ist. Der Nadelträger mit der Injektionsnadel ist in den Nadelhalter eingeschraubt und so mit diesem verbunden. Dadurch ist es möglich, unterschiedliche Nadelgrößen mittels des Nadelträgers in den vormontierten Nadelhalter im Spritzenzylinder einzusetzen. Weiters sind Kupplungsmittel zum Verbinden des Kolbenstopfens bzw. der Kolbenstange mit dem Nadelhalter der Nadelbaugruppe vorgesehen, um die gesamte Nadelbaugruppe nach der Aufnahme und kurzfristigen Abgabe der Injektionslösung aus Sicherheitsgründen in den Spritzenzylinder zurückziehen zu können. Der Innenraum zur Aufnahme der Injektionslösung wird dabei durch den Spritzenzylinder, den Nadelhalter der Baugruppe sowie dem davon distanzierten Kolbenstopfen gebildet. Die Injektionsnadel steht dabei nur über Kanäle im Nadelträger bzw. Nadelhalter mit dem Innenraum der Injektionsspritze in Strömungsverbindung.

Die US 6,613,016 B1 offenbart ebenfalls eine Injektionsspritze für den Einmalgebrauch, bei welcher die Injektionslösung für die Abgabe kurzfristig in den Spritzenzylinder eingezogen wird. Diese Injektionsspritze umfasst einen Spritzenzylinder, einen durch eine Kolbenstange im Spritzenzylinder verschiebbaren Kolbenstopfen sowie einer im Spritzenzylinder aufgenommenen Nadelbaugruppe. Diese Nadelbaugruppe umfasst einen an der Innenwandung des Spritzenzylinders anliegenden Verbindungsteil. Dieser Verbindungsteil dient zum Aufnehmen eines Nadelträgers, an dem die Injektionsnadel gehaltert ist. Durch die zusätzliche Anordnung des Verbindungsstücks ist es wiederum möglich, den Nadelträger mitsamt der Nadel erst nachträglich an der Injektionsspritze anzuordnen, um dadurch möglichen Stichverletzungen vorzubeugen. Um nach erfolgter Abgabe der Injektionslösung die gesamte Nadelbaugruppe in den Spritzenzylinder zurückziehen zu können, sind zwischen der Kolbenstange und dem Verbindungsteil Kupplüngsmittel vorgesehen. Die Injektionsnadel steht dabei über Kanäle des Nadelträgers sowie Verbindungsstücks mit dem Innenraum zur Aufnahme der Injektionslösung in Strömungsverbindung.

Aus der EP 1 514 566 A1 ist ebenfalls eine Injektionsspritze für den Einmalgebrauch bekannt geworden, die einen Spritzenzylinder, einen durch eine Kolbenstange im Spritzenzylinder verschiebbaren Kolbenstopfen sowie eine im Spritzenzylinder aufgenommene Nadelbaugruppe umfasst. Auch diese Injektionsspritze dient zur kurzfristigen Aufnahme und Abgabe von einer Injektionslösung, wobei der Innenraum zur Aufnahme derselben, durch den Spritzenzylinder, die Nadelbaugruppe sowie den davon distanzierten Kolbenstopfen gebildet wird. Die Nadelbaugruppe umfasst einen eigenen Nadelträger, an dem die Injektionsnadel gehaltert ist. Im vorderen Endbereich des Spritzenzylinders ist ein rohrförmig ausgebildetes Zwischenstück als Halteelement für den Nadelträger angeordnet. Dieses Zwischenstück umfasst einen Tragkörper sowie ein daran aus Gummi gebildetes Halteelement. Dieses Halteelement dient einerseits zur Halterung des Nadelträgers und andererseits als dichtendes Abschlusselement hin zur Innenwand des Spritzenzylinders. Die Injektionsnadel steht über eigene Kanäle im Nadelträger bzw. den vom gummiförmigen Halteelement ausgebildeten Zwischenraum mit dem Innenraum zur Aufnahme der Injektionslösung in Strömungsverbindung.

Die US 5,263,934 A offenbart weiters eine Injektionsspritze für den Einmalgebrauch zur kurzfristigen Aufnahme und Abgabe einer Injektionslösung. Die Injektionsspritze umfasst einen Spritzenzylinder, einen durch eine Kolbenstange im Spritzenzylinder verschiebbaren Kolbenstopfen sowie eine im Spritzenzylinder aufgenommene Nadelbaugruppe. Die Injektionsnadel ist in einem eigenen Nadelträger gehalten, der an der Innenwandung des Spritzenzylinders zur Anlage kommt. Zur besseren Führung im vordersten Teil des Spritzenzylinders ist ein eigenes buchsenformiges Führungselement vorgesehen, welches von der Injektionsnadel durchragt ist. Zwischen dem Nadelhalter der Nadelbaugruppe und dem Kolbenstopfen sind Kupplungsmittel vorgesehen, um nach erfolgter Abgabe der Injektionslösung die Nadelbaugruppe in den Spritzenzylinder zurückziehen zu können.

Schließlich beschreibt die WO 91/08788 A1 eine Injektionsspritze für den Einmalgebrauch, die aus einem Spritzenzylinder, einem durch eine Kolbenstange im Spritzenzylinder verschiebbaren Kolbenstopfen sowie einer im Stopfenzylinder aufgenommenen Nadelbaugruppe umfasst. In der Ausgangsstellung ist die Nadelbaugruppe über deren Nadelhalter mit der Kolbenstange gekuppelt und in einer im Spritzenzylinder zurückgezogenen Position angeordnet. Vor dem Aufnehmen der Injektionslösung in den Innenraum ist die Kolbenstange mitsamt der daran angeordneten Nadelbaugruppe in Längsrichtung des Spritzenzylinders zu verschieben, sodass der Nadelträger im vorderen Bereich des Spritzenzylinders an diesem verrastend gehalten werden kann. Anschließend daran wird die Kupplung zwischen der Kolbenstange und dem Nadelträger gelöst, sodass über die Injektionsnadel die Lösung im Zusammenwirken mit dem Zurückziehen der Kolbenstange und des Kolbenstopfens in den Innenraum eingesaugt wird. Nach erfolgter Abgabe wird die Kolbenstange erneut mit dem Nadelträger gekuppelt und nach entsprechender Lösung der Verriegelung zwischen dem Nadelträger und dem Spritzenzylinder kann die gesamte Nadelbaugruppe wiederum mit der Kolbenstange in den Innenraum zurückbewegt werden. Die Injektionsnadel steht über einen eigenen, im Nadelträger angeordneten Kanal mit dem Innenraum zur Aufnahme der Injektionslösung in Strömungsverbindung.

Injektionsspritzen, bei denen nach erfolgter Injektion die Nadel in den Spritzenzylinder zurückgezogen wird, um Verletzungen durch die Injektionsnadel zu vermeiden und den erneuten Gebrauch der Spritze zu verhindern, sind in vielerlei Ausführungen bekannt, so zum Beispiel aus dem Dokument US 6,613,016 B1. So lange bei derartigen Spritzen die Injektionslösung kurz vor der beabsichtigten Injektion aufgezogen wird, besteht hinsichtlich der Werkstoffwahl kein Problem. Hingegen sind derzeit nur drei Werkstoffe bekannt, die für den längerfristigen Kontakt mit einer Injektionslösung zugelassen sind. Dies sind Glas, Pharmagummi und nichtrostender Stahl. Durch diese Einschränkung bei der Werkstoffwahl ergeben sich bei zum Einmalgebrauch bestimmten Spritzen, die vorgefüllt gelagert werden und bei denen die Nadel nach der Injektion in den Spritzenzylinder zurückgezogen wird, konstruktive Probleme, da die für alle Arten von Wegwerfartikeln gebräuchlichen, durch Spritzgießen verarbeitbaren Kunststoffe während der Lagerung der Spritze nicht mit der Injektionslösung in Kontakt kommen dürfen.

Ausgehend von diesem Stand der Technik liegt der Erfindung die Aufgabe zugrunde, eine Spritze der eingangs genannten Art vorzuschlagen, bei der die Injektionslösung während der Lagerung ausschließlich mit den genannten derzeit zugelassenen Werkstoffen in Kontakt steht.

Diese Aufgabe wird erfindungsgemäß dadurch gelöst, dass der Innenraum im Ausgangszu stand mit der Injektionslösung vorgefüllt ist, wobei auf ein dem Innenraum zugewendetes hinteres Ende des Nadelhalters der aus Pharmagummi bestehende Dichteinsatz aufgesetzt ist und dieser den Nadelhalter gegen den die Injektionslösung aufnehmenden Innenraum hin vollständig abdeckt und der Dichteinsatz vom hinteren Ende der im Nadelhalter gehaltenen Injektionsnadel hin zum Innenraum durchragt ist und dass der Nadelhalter an einem Bereich seines Außenmantels mit Längsrippen ausgestattet ist und diese in einem verengten Endbereich des Spritzenzylinders aufgenommen sind sowie an diesem anliegen und dass an einem vorderen Ende eines Kragens des Dichteinsatzes ein Dichtwulst angeordnet ist und der Dichtwulst dichtend an der Innenwand des Spritzenzylinders anliegt und diesen bakteriendicht verschließt.

Der sich durch die Merkmale des Kennzeichenteiles des Anspruches 1 ergebende Vorteil besteht insbesondere darin, dass Trotz der Verwendung von Kunststoff für den Nadelhalter die Injektionslösung während der Lagerung der Spritze nicht mit nicht zugelassenen Werkstoffen in Kontakt kommt. Da der Spritzenzylinder vorzugsweise aus Glas besteht und sich folglich nicht in sehr engen Toleranzen herstellen lässt, nehmen die Längsrippen ein gegebenenfalls vorhandenes Übermaß auf, indem sie sich beim Einsetzen des Nadelhalters in den Spritzenzylinder leicht verformen. Ein weiterer Vorteil dieser erfindungsgemäßen Lösung besteht in der einfachen und kostengünstigen Herstellung und Montage der Spritze, was hinsichtlich des einmaligen Gebrauchs besonders wichtig ist.

Vorteilhaft ist auch eine weitere Ausführungsform nach Anspruch 2, gemäß welcher der Nadelhalter an seinem injektionsseitigen Ende einen im Durchmesser erweiterten Kopf aufweist, auf den eine Nadelschutzkappe aufsetzbar ist. Durch diese Maßnahme kann der Durchmesser der Nadelschutzkappe geringer gehalten werden, als wenn die Nadelschutzkappe auf den Spritzenzylinder aufsetzbar wäre.

Durch die Ausbildung nach Anspruch 3, die dadurch gekennzeichnet ist, dass der Nadelhalter an seinem von der Injektionsseite abgewandten Ende eine stirnseitige Ausnehmung hat, in welche das von der Injektionsseite abgewandte Ende der Injektionsnadel ragt, werden günstige Voraussetzungen geschaffen, um eine vormontierte Nadelbaugruppe zu bilden.

Nach einer anderen Ausführungsvariante gemäß Anspruch 4 ist in der die Ausnehmung umgebenden Wandung des Nadelhalters mindestens ein im Wesentlichen axial verlaufender Schlitz angeordnet. Dadurch erhält der betreffende Bereich eine radiale Elastizität zum Aufnehmen der Kupplungsmittel der Kolbenstange.

Eine andere, im Anspruch 5 definierte Ausführungsart sieht vor, dass der Dichteinsatz sich mit einem Fortsatz in die Ausnehmung des Nadelhalters erstreckt und den Außenmantel des Nadelhalters im Bereich der Ausnehmung mit einem Kragen umschließt. Dadurch ist einerseits der Dichteinsatz am Nadelhalter gehalten und andererseits eine Abdichtung der Nadelbaugruppe im Spritzenzylinder gewährleistet.

Die weitere Ausführungsart nach Anspruch 6, bei welcher der Dichteinsatz im von der Injektionsseite abgewandten Endbereich seines Fortsatzes einen Ringwulst hat, der in eine in der Innenwand der Ausnehmung vorgesehene Ringnut greift, erlaubt eine Verrastung des Dichteinsatzes mit dem Nadelhalter.

Die Ausführungsart nach Anspruch 7 sieht vor, dass der am Kragen des Dichteinsatzes angeordnete Dichtwulst O-Ring ähnlich ausgebildet ist und in eine am Nadelhalter angeordnete Ringnut eingreift. Dadurch erfolgt unabhängig von Durchmesser-Toleranzschwankungen des Spritzenzylinders eine sichere Abdichtung der Nadelbaugruppe.

Wenn gemäß der Ausführungsart nach Anspruch 8 am Außenumfang des Dichteinsatzes ein Ansatz gebildet ist, mit dem der Dichteinsatz an einer im injektionsseitigen Bereich des Spritzenzylinders gebildeten Schulter axial anliegt, werden die während der Injektion auf die Nadelbaügruppe ausgeübten Axialkräfte optimal aufgenommen.

Durch die Ausrührungsart nach Anspruch 9, welche vorsieht, dass im Dichteinsatz ein Hohlraum vorhanden ist, von welchem aus sich eine Öffnung unter Bildung eines Ringbunds zum Innenraum des Spritzenzylinders erstreckt, wobei sich das hintere Ende der Injektionsnadel in diesen Hohlraum erstreckt, ist die Nadelbaugruppe nach erfolgter Injektion sicher mit der Kolbenstange kuppelbar.

Nach einer weiteren Ausführungsart nach Anspruch 10 ist nahe bei dem von der Injektionsseite abgewandten Ende des Spritzenzylinders ein innerer Ringwulst angeordnet, dessen vordere, der Injektionsseite zugewandte Flanke mindestens annähernd rechtwinklig zur Wand des Spritzenzylinders ausgerichtet ist und dessen hintere, der Injektionsseite abgewandte Flanke mit der Wand des Spritzenzylinders einen stumpfen Winkel einschließt. Dadurch ist ein Positioniermittel geschaffen, welches beim Montieren der Kolbenstange eine Rasthilfe bietet und beim Zurückziehen der Kolbenstange als Anschlag dient.

Eine andere, im Anspruch 11 umschriebene Ausführungsart sieht vor, dass an der Kolbenstange Anschlagnocken vorhanden sind, die eine von der Injektionsseite abgewandte, im Wesentlichen rechtwinklig zur Längsachse der Kolbenstange ausgerichtete Flanke aufweisen, die beim Zurückziehen der Kolbenstange an der vorderen, der Injektionsseite zugewandten Flanke des inneren Ringwulstes anstehen und dadurch verhindern, dass die Kolbenstange ganz aus dem Spritzenzylinder herausgezogen werden kann.

Die Ausführungsart nach Anspruch 12 zeichnet sich dadurch aus, dass an der Kolbenstange in einem Abstand von den Anschlagnocken auf der von der Injektionsseite abgewandten Seite Rastnocken angeordnet sind, deren Flanken so ausgebildet sind, dass die Rastnocken unter Überwindung eines Widerstands in beiden Richtungen am inneren Ringwulst des Spritzenzylinders vorbei bewegt werden können.

Wenn nach der Ausführungsart gemäß Anspruch 13 die Kolbenstange im Bereich der Rastnocken Ausnehmungen aufweist, damit die Rastnocken radial federnd nachgeben können, wird der genannte Widerstand so gering gehalten, dass bei dessen Überwindung keine Beschädigungen auftreten können.

Eine weitere Ausführungsart nach Anspruch 14 zeichnet sich dadurch aus, dass an der Kolbenstange in einem Bereich zwischen den Anschlagnocken und den Rastnocken eine Sollbruchstelle vorhanden ist. Dadurch kann nach erfolgter Injektion und dem Zurückziehen der Kolbenstange mitsamt der daran angekuppelten Nadelbaugruppe der aus dem Spritzenzylinder heraus ragende Teil der Kolbenstange abgebrochen werden, wodurch zuverlässig verhindert wird, dass die Spritze noch einmal benutzt wird.

Nach einer alternativen Ausführungsart gemäß Anspruch 15 besteht die Kolbenstange aus zwei Teilen, die durch weitere Kupplungsmittel lösbar miteinander verbunden sind, wobei die Kupplungsmittel derart ausgebildet sind, dass sie ausschließlich in einer Betriebslage lösbar sind, in der die Kolbenstange bis zu einem Anschlag zurück gezogen ist. Dadurch kann nach erfolgter Injektion und dem Zurückziehen der Kolbenstange mitsamt der daran angekuppelten Nadelbaugruppe der aus dem Spritzenzylinder heraus ragende Teil der Kolbenstange abgekuppelt werden, wodurch zuverlässig verhindert wird, dass die Spritze noch einmal benutzt wird.

Eine besondere Ausführungsart nach Anspruch 16 sieht vor, dass die Kupplungsmittel eine mit dem Kolbenstopfen verbundene Kupplungshülse enthalten, an der von der Injektionsseite abgewandte Kupplungsklauen angeformt sind, welche einen Kupplungskopf hintergreifen, wobei der Kupplungskopf nur dann durch die radial federnd nachgiebigen Halteklauen freigebbar ist, wenn diese sich auf mindestens einem Teil ihrer Länge außerhalb des Spritzenzylinders oder in einem Endbereich des Spritzenzylinders mit vergrößertem Durchmesser befinden. Dadurch kann die gekuppelte Kolbenstange mitsamt der daran angekuppelten Nadelbaugruppe nach erfolgter Injektion zuverlässig bis zu einem Endanschlag zurückgezogen werden, bevor der dann der aus dem Spritzenzylinder herausragende Teil der Kolbenstange wie oben beschrieben abgekuppelt wird.

Schließlich ist gemäß einer weiteren Ausführung nach Anspruch 17 am von der Injektionsseite abgewandten hinteren Ende der Kolbenstange eine Endhülse angeordnet, deren nach hinten gerichtete Öffnung einen auf das vordere Ende des Spritzenzylinders passenden Durchmesser aufweist. So kann die Spritze nach Gebrauch wieder dicht verschlossen werden.

Ein Ausführungsbeispiel der Erfindung wird nachstehend unter Bezugnahme auf die angefügten Zeichnungen näher beschrieben.

Es zeigt:
- Fig. 1: eine vorgefüllte Injektionsspritze zum Einmalgebrauch in der Ausgangsposition;
- Fig. 2: die Spritze im injektionsbereiten Zustand;
- Fig. 3: die Spritze nach der Injektion;
- Fig. 4: die Spritze mit zurückgezogener Injektionsnadel;
- Fig. 5: die Spritze in gesichertem Zustand, bereit zur Entsorgung;
- Fig. 6: eine Einzelheit aus Fig. 4 in vergrößertem Maßstab;
- Fig. 7: die Nadelbaugruppe der Spritze gemäß den Figuren 1 bis 6;
- Fig. 8: den Nadelhalter inklusive Injektionsnadel der Nadelbaugruppe gemäß Fig. 7;
- Fig. 9: den Dichteinsatz der Nadelbaugruppe gemäß Fig. 7;
- Fig. 10: eine andere Ausführungsart der Spritze in der Ausgangsposition;
- Fig. 11: eine weitere Ausführungsart der Spritze in der Ausgangsposition;
- Fig. 12: die Spritze gemäß Fig. 11 nach erfolgter Injektion und
- Fig. 13: eine Einzelheit aus Fig. 12 in vergrößertem Maßstab.

Einführend sei festgehalten, dass in den unterschiedlich beschriebenen Ausführungsformen gleiche Teile mit gleichen Bezugszeichen bzw. gleichen Bauteilbezeichnungen versehen sind, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. vorne, hinten seitlich usw. auf die unmittelbar beschriebene sowie dargestellte Figur bezogen und sind bei einer Lageänderung sinngemäß auf die neue Lage zu übertragen.

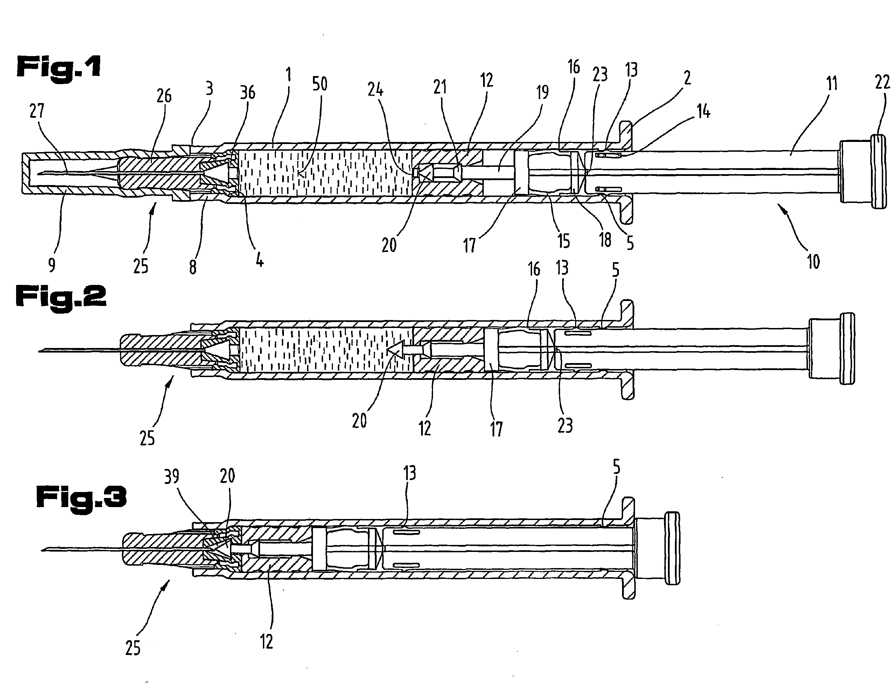

Die Figuren 1 bis 5 zeigen fünf Schritte bei der Bedienung eines Ausführungsbeispiels der erfindungsgemäßen Spritze, bei welcher es sich vorwiegend um eine vorbefüllbare Spritze für den Einmalgebrauch handelt.

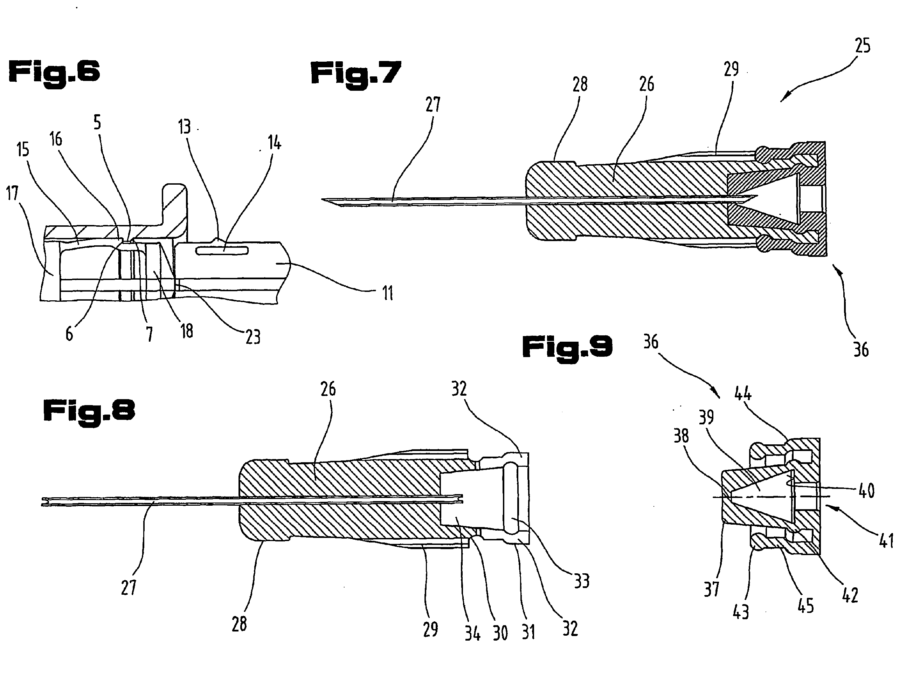

Ein Spritzenzylinder 1 aus Glas weist an seinem in Injektionsrichtung hinteren Ende einen Flansch 2 auf, der währen der Injektion als Auflage für die Finger einer die Spritze bedienenden Person dient. In dieser Beschreibung ist generell mit "vorne" die Injektionsseite und mit "hinten" die der Injektionsnadel abgewandte Seite der Spritze gemeint. An seinem vorderen Ende ist am Spritzenzylinder 1 ein gegenüber diesem im Durchmesser verjüngter Hals 8 ausgebildet, an dem mindestens ein Arretierungswulst 3 angeformt ist, dessen Funktion weiter unten erläutert wird. Im Inneren des Spritzenzylinders 1 ist am Übergang zum Hals 8 eine Schulter 4 gebildet. Im Inneren des Spritzenzylinders 1 ist nahe bei dessen hinterem Ende ein Ringwulst 5 ausgebildet. Wie der vergrößerte Ausschnitt gemäß Fig. 6 zeigt, hat dieser Ringwulst eine vordere, im Wesentlichen rechtwinklig zur Innenwand des Spritzenzylinders 1 ausgerichtete Flanke 6 und eine hintere Flanke 7, die zur Innenwand des Spritzenzylinders 1 einen stumpfen Winkel aufweist. Im vorderen Ende des Spritzenzylinders 1 ist eine als Ganzes mit 25 bezeichnete und weiter unten näher beschriebene Nadelbaugruppe 25 aufgenommen, auf welcher in der Betriebslage gemäß Fig. 1 eine Nadelschutzkappe 9 sitzt.

Im hinteren Ende des Spritzenzylinders 1 ist eine als Ganzes mit 10 bezeichnete Kolbenbaugruppe 10 eingesetzt, die ihrerseits eine in der Betriebslage nach Fig. 1 aus dem Spritzenzylinder 1 heraus ragende Kolbenstange 11 und einen auf deren vorderem Ende sitzenden Kolbenstopfen 12 umfasst. Die Kolbenstange 11 besteht vorzugsweise wie dargestellt aus zwei sich in einem rechten Winkel kreuzenden Stegen, von denen mindestens einer einen Rastnocken 13 trägt, der in der Betriebslage nach Fig. 1 am Ringwulst 5 ansteht und so einen Widerstand gegen das Bewegen der Kolbenstange 11 in Injektionsrichtung bildet. Damit der Rastnocken 13 radial nach innen nachgeben kann, ist unter diesem im Steg eine Ausnehmung 14 angeordnet. Vor dem oder den Rastnocken 13 ist an der Kolbenstange 11 eine Sollbruchstelle 23 und daran nach vorne anschließend eine hintere Platte 18 und eine vordere Platte 17 angeordnet. Im Bereich zwischen diesen Platten sind die Stege so ausgespart, dass außen liegende, radial nach innen federnd nachgiebige Federstege 15 gebildet sind, an denen radial nach außen ragende Anschlagnocken 16 ausgebildet sind. In der Fortsetzung auf der vorderen Seite der vorderen Platte 17 besteht die Kolbenstange 11 aus einem vorzugsweise zylindrischen Schaft 19, der einen radial vorstehenden Ansatz 21 und schließlich am Ende eine pfeilförmige Spitze 20 trägt. Im Bereich der Spitze 20 ist der Kolbenstopfen 12 durch eine einstückig mit ihm ausgebildete Membrane 24 dicht verschlossen. Am hinteren Ende ist die Kolbenstange 11 mit einer nach hinten offenen Endhülse 22 abgeschlossen.

Die bereits erwähnte Nadelbaugruppe 25 ist in den Figuren 7 bis 9 in einem gegenüber den Figuren 1 bis 5 vergrößerten Maßstab genauer dargestellt. Eine Injektionsnadel 27 aus nichtrostendem Stahl ist von einem aus Kunststoff bestehenden Nadelhalter 26 umschlossen. Vorzugsweise ist diese in Fig. 8 in einem Längsschnitt dargestellte Kombination von Injektionsnadel 27 und Nadelhalter 26 durch Umspritzen der Nadel in einer Spritzgießmaschine hergestellt. Am vorderen Ende weist der Nadelhalter 26 einen im Durchmesser erweiterten Kopf 28 auf, auf welchem in der Betriebslage gemäß Fig. 1 die Nadelschutzkappe 9 sitzt. An der vom Kopf 28 entfernten Seite weist der Nadelhalter an seinem Außenumfang angeordnete Längsrippen 29 auf, welche bei der Aufnahme der Nadelbaugruppe 25 im Hals 8 des Spritzenzylinders 1 die bei der Herstellung von Glasteilen unvermeidlichen Toleranzen ausgleichen. Anschließend an die Längsrippen 29 ist im Nadelhalter 26 eine Ringnut 30 eingearbeitet und dieser benachbart befindet sich ein Bund 31. Ausgehend vom hinteren Ende des Nadelhalters 26 sind in diesem zwei um 180° versetzte Schlitze 32 vorhanden, die sich bis in den Bereich der Ringnut 30 erstrecken. Diese Schlitze 32 sind nur in Fig. 8, sichtbar, weil der Nadelhalter 26 in Fig. 7 gegenüber der Darstellung in Fig. 8 um 90° um die Längsachse gedreht ist. Eine in etwa kegelstumpfförmige Ausnehmung 34 erstreckt sich vom hinteren Ende des Nadelhalters 26 koaxial in diesen hinein, wobei das hintere Ende der Injektionsnadel 27 in diese Ausnehmung hinein ragt, wie dies in Fig. 8 deutlich zu sehen ist. In dieser Ausnehmung 34 ist eine innere Ringnut 33 eingearbeitet.

Auf das hintere Ende des Nadelhalters 26 ist ein Dichteinsatz 36 aufgesetzt, wie er in Fig. 9 dargestellt ist. Dieser weist einen in die Ausnehmung 34 passenden Fortsatz 37 auf, der von einem umlaufenden Kragen 45 umgeben ist. Im Inneren des Dichteinsatzes 36 befindet sich ein kegelförmiger Hohlraum 39, von welchem aus sich eine zylindrische Öffnung 41 unter Bildung eines Ringbunds 40 nach hinten erstreckt. Im Bereich der Spitze der kegelförmigen Ausnehmung 39 ist in der Wandung des Dichteinsatzes eine Schwächung 38 vorgesehen, welche beim Montieren des Dichteinsatzes 36 auf dem Nadelhalter 26 vom hinteren Ende der Injektionsnadel 26 durchstochen wird. In Fig. 7 ist der montierte Zustand sichtbar, in welchem das hintere Ende der Injektionsnadel 27 in die kegelförmige Ausnehmung 39 des Dichteinsatzes 36 ragt. Ein am hinteren Ende des Fortsatzes 37 vorgesehener Ringwulst 42 greift bei montiertem Dichteinsatz 36 in die innere Ringnut 33 des Nadelhalters 26 ein und sichert und positioniert den Dichteinsatz 36 im Nadelhalter 26. Am vorderen Ende des Kragens 45 ist ein Dichtwulst 43 angeordnet, welcher die Nadelbaugruppe 25 ähnlich wie ein O-Ring im Spritzenzylinder 1 abdichtet und diesen bakteriendicht verschließt. Im Abstand vom Dichtwulst 43 ist ein Absatz 44 angeordnet, der bei der Montage der Nadelbaugruppe 24 im Spritzenzylinder 1 an der Schulter 4 des Spritzenzylinders ansteht und somit einen Anschlag bildet.

Durch die beschriebene Konstruktion steht die in der Spritze im Ausgangszustand gemäß Fig. 1 vorhandene Injektionslösung 50 nirgends mit Kunststoff oder einem anderen für länger dauernden Kontakt mit der Injektionslösung 50 nicht zugelassenen Werkstoff in Kontakt. Der die Nadelbaugruppe 25 zum Inneren des Spritzenzylinders 1 abschließende Dichteinsatz 36 besteht aus Pharmagummi, die den Dichteinsatz 36 durchdringende und dadurch auch mit der Injektionslösung 50 in Kontakt stehende Injektionsnadel 27 besteht wie bereits erwähnt aus nichtrostendem Stahl. Der den Spritzenzylinder 1 hinten dicht abschließende Kolbenstopfen 12 besteht ebenfalls aus Pharmagummi und der Spritzenzylinder 1 besteht wie erwähnt aus Glas. Als nichtrostender Stahl für die hier beschriebenen Zwecke eignet sich beispielsweise der unter der Bezeichnung Niro 1.43.01 bekannte Werkstoff. Als so genannter Pharmagummi eignet sich beispielsweise der unter der Bezeichnung FM 257/2 bekannte Werkstoff.

Bei der Montage der Spritze wird zuerst die vormontierte Nadelbaugruppe 25 von hinten in den Spritzenzylinder 1 eingeführt und vorgeschoben, bis der Absatz 44 des Dichteinsatzes 36 an der Schulter 4 ansteht. Dann wird die Nadelschutzkappe 9 auf den Nadelhalter 26 aufgesteckt, wobei es zur Abdichtung der Nadelspitze vorteilhaft ist, wenn die Nadelspitze in die Stirnwand der Nadelschutzkappe 9 soweit einsticht, dass ein Auslaufen der nachträglich eingefüllten Injektionslösung 50 verhindert wird. Damit ist die Spritze injektionsseitig bakteriendicht verschlossen. Dann wird die Injektionslösung 50 eingefüllt und der Kolbenstopfen 12 bis zu der in Fig. 1 und 2 dargestellten Position in den Spritzenzylinder 1 geschoben. Danach wird die Kolbenstange 11 eingeschoben, wobei die am Ringwulst 5 anstehenden Rastnocken 13 verhindern, dass die Kolbenstange 11 so weit eingeschoben wird, bis die Spitze 20 die Membrane 24 des Kolbenstopfens durchdringt.

Die Benutzung der Spritze läuft folgendermaßen ab. Ausgehend von der Ausgangsposition gemäß Fig. 1 wird zuerst die Nadelschutzkappe 9 entfernt, die Spritze entlüftet und die Injektionsnadel 27 wird in die Haut bzw. das Gewebe einer Person eingestochen.

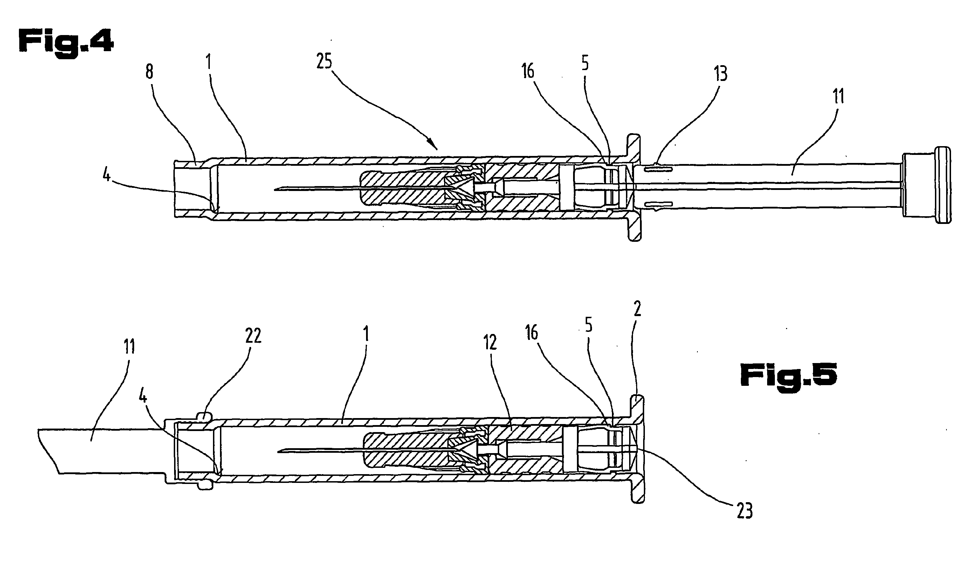

In der Betriebslage gemäß Fig. 2 wurde die Kolbenstange 11 nach vorne bewegt, wobei der Kolbenstopfen 12 zunächst noch an seinem Platz im Spritzenzylinder 1 verbleibt, an dem er durch Reibung und den Druck der inkompressiblen Injektionslösung 50 festgehalten wird. Nach dem Überwinden des durch die Rastnocken 13 und den Ringwulst 5 verursachten Widerstandes kann die Kolbenstange 11 nach vorne verschoben werden, wobei die aus Kunststoff bestehende Spitze 20 der Kolbenstange 11 die Membrane 24 des Kolbenstopfens 12 durchdringt, wobei sich der zurückfedernde Werkstoff des Kolbenstopfens 12 dichtend an den Schaft anlegt und so verhindert, dass Injektionslösung nach hinten austreten kann. Der Kontakt der Spitze 20 mit der Injektionslösung besteht bis zu deren vollständigem Ausspritzen in der Regel nur wenige Sekunden. Wie man in Fig. 4 sieht, steht nun die vordere Platte 17 am hinteren Ende des Kolbenstopfens 12 an und dieser wird im Zuge der weiteren Bewegung der Kolbenstange im Spritzenzylinder 1 nach vorne geschoben und stößt die Injektionslösung 50 durch die Injektionsnadel 27 aus.

In der Betriebslage nach Fig. 3 ist die Injektionslösung vollständig ausgestoßen und die Spitze 20 der Kolbenstange 11 ist in die Ausnehmung 39 im Dichteinsatz der 36 Nadelbaugruppe 25 eingedrungen. Dieses Eindringen wurde dadurch ermöglicht beziehungsweise erleichtert, dass sich das hintere Ende des Nadelhalters 26 bedingt durch die Schlitze 32 radial dehnen lässt.

Ausgehend von der Betriebslage gemäß Fig. 3 wird zuerst die ganze Spritze zurückgezogen, so dass die Spitze der Injektionsnadel 27 die Haut der Person verlässt. Dann wird die Kolbenstange in Bezug auf den Spritzenzylinder 1 zurückgezogen, wobei der Ringbund 40 verhindert, dass die Spitze 20 die Ausnehmung 39 wieder verlässt. Folglich wird die gesamte Nadelbaugruppe 25 nach hinten in den Spritzenzylinder hinein gezogen. Da sowohl die nach vorne gerichteten als auch die nach hinten gerichteten Flanken der Rastnocken 13 mit der Kolbenstange 11 einen stumpfen Winkel einschließen, werden die Rastnocken 13 durch den Ringwulst 5 nach innen gedrückt und können diesen passieren. Die in Fig. 4 dargestellte Endlage dieser Rückzugsbewegung wird durch Anstehen der im Wesentlichen rechtwinklig zur Längsachse orientierten hinteren Flanke der Anschlagnocken 16 am Ringwulst 5 beendet. Nun wird die Kolbenstange 11 abgebrochen, was durch die Sollbruchstelle 23 erleichtert wird. Das abgebrochene Ende der Kolbenstange 11 kann mit der Endhülse 22 auf den Hals 8 des Spritzenzylinders 1 aufgesetzt werden und wird in dieser Lage durch innere rund umlaufende Rillen am Arretierungswulst 3 des Spritzenzylinders festgehalten. Damit ist die Injektionsnadel 27 vollständig eingeschlossen und vor Berührung geschützt. Es liegt auf der Hand, dass in kinematischer Umkehr am hinteren Ende der Kolbenstange 11 auch ein zylindrischer Zapfen vorgesehen sein kann (nicht zeichnerisch dargestellt), der nach dem Abbrechen des hinteren Teils der Kolbenstange in den Hals 8 des Spritzenzylinders 1 gesteckt werden kann.

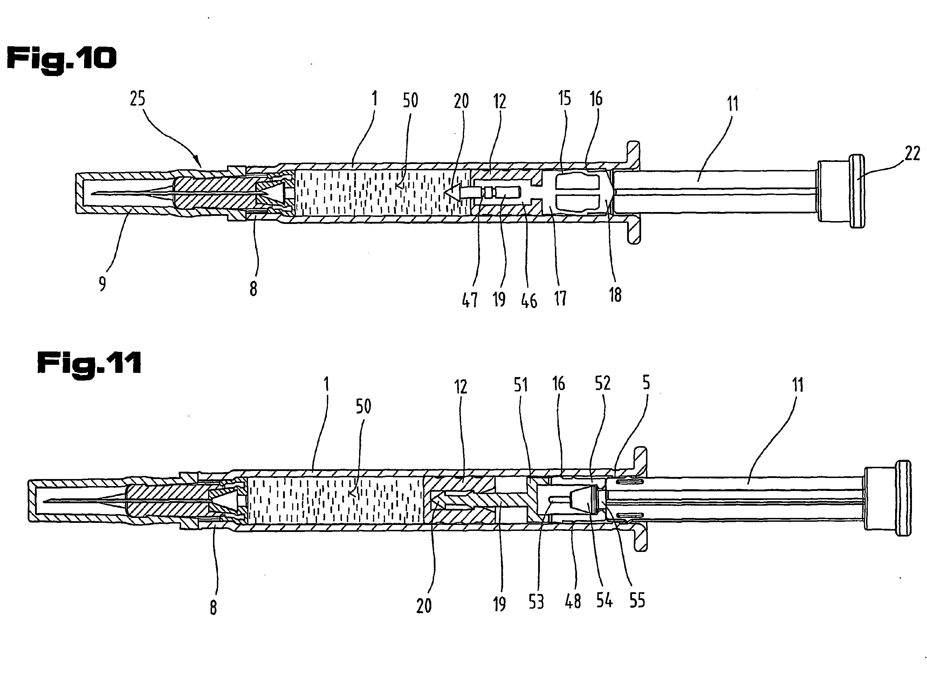

Fig. 10 zeigt ein weiteres Ausführungsbeispiel der erfindungsgemäßen Injektionsspritze. Im Gegensatz zum Beispiel gemäß Fig. 1 bis 5 ragt bei diesem Beispiel die Spitze 20 bereits in der Ausgangsposition aus dem Kolbenstopfen 12 und steht daher mit der Injektionslösung 50 in Kontakt. Dies bedingt, dass die Spitze 20 aus einem zugelassenen Werkstoff besteht. Im vorliegenden Beispiel ist dies nichtrostender Stahl, beispielsweise vom weiter oben genannten Typ. Wie man in Fig. 10 sieht, ist die Spitze 20 mit ihrem Schaft in einem Aufnahmezylinder 46 eingegossen, beziehungsweise wurde der Schaft 19 durch Umspritzen in den Aufnahmezylinder 46 integriert, welcher seinerseits mit der Kolbenstange 11 einstückig ausgebildet ist. Damit der Schaft 19 im Aufnahmezylinder 46 gut verankert ist und die beim Zurückziehen der Kolbenstange 11 auftretenden Zugkräfte nicht zur Trennung des Schafts 19 vom Aufnahmezylinder 46 führen, sind im Schaft 19 Ringnuten 47 vorgesehen, die beim Spritzgießen de Aufnahmezylinders 46 mit Kunststoff gefüllt werden. Das beim ersten beschriebenen Ausführungsbeispiel in der Betriebslage gemäß Fig. 1 sichtbare Spiel zwischen dem Kolbenstopfen 12 und der vorderen Platte 17 ist im Beispiel gemäß Fig. 10 nicht vorhanden und bei der Montage der Spritze kann nach dem Befüllen mit der Injektionslösung 50 die gesamte, aus der Kolbenstange 11, dem in diese eingesetzten Schaft 19 aus nichtrostendem Stahl und dem auf letzteren aufgesetzten Kolbenstopfen 12 bestehende Kolbenbaugruppe eingesetzt werden.

In Fig. 11 bis 13 ist ein weiteres Ausführungsbeispiel der Injektionsspritze dargestellt, bei welchem der Kolbenstopfen 12, der Schaft 19 und die Spitze 20 gleich aufgebaut sind, wie bei dem an Hand der Fig. 1 bis 5 beschriebenen Ausführungsbeispiel. Das Beispiel gemäß Fig. 11 bis 13 unterscheidet sich von den vorangehend beschriebenen Beispielen dadurch, dass die Kolbenstange keine Sollbruchstelle 23 aufweist, sondern zweiteilig ausgebildet ist.

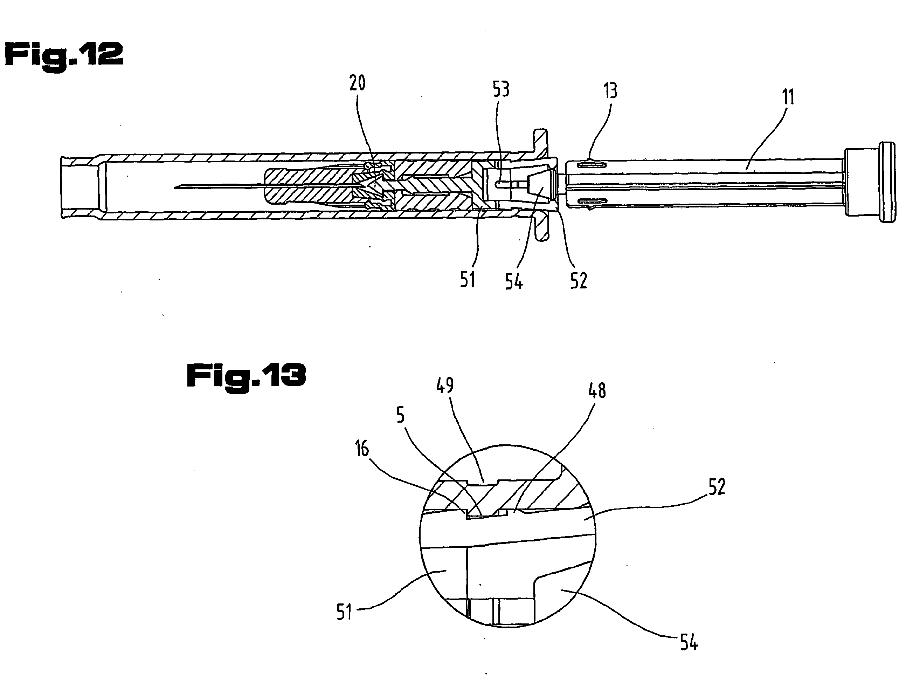

Am hinteren Ende des Schafts 19 ist einstückig eine Kupplungshülse 51 angeformt, deren Wandung mehrere am hinteren Ende der Kupplungshülse 51 offene Einschnitte 53 aufweist, wodurch Halteklauen 52 gebildet sind, die einerseits die Funktion der zuvor beschriebenen Federstege 15 übernehmen, andererseits nach dem Zurückziehen der Kolbenstange 11 mit der angekuppelten Nadelbaugruppe 25 die Trennung der beiden Teile der Kolbenstange ermöglicht, wie dies nachstehend beschrieben wird. Am hinteren Teil der Kolbenstange 11 ist ein Kupplungskopf 54 angeformt, der in die Kupplungshülse 51 ragt und dort durch die Halteklauen 52 axial festgehalten wird, indem diese in einen hinter dem Kupplungskopf 54 vorgesehene Hinterschnitt 55 greifen. Dadurch wird es möglich, nach erfolgter Injektion die Kolbenstange 11 und die Nadelbaugruppe 25 zurückzuziehen. So lange sich die Kupplungshülse 51 im Spritzenzylinder 1 befindet, können die Halteklauen 52 nicht so weit radial nach außen federnd nachgeben, dass sie den Kupplungskopf 54 freigeben. Erst wenn die Kupplungshülse die in Fig. 12 dargestellte Position erreicht und mindestens die freien Enden der Halteklauen 52 den Spritzenzylinder 1 verlassen haben, können die Halteklauen 52 so weit radial nach außen federnd nachgeben, dass der Kupplungskopf 54 frei gegeben wird. Kurz vor Erreichen dieser Position musste ein außen an den Halteklauen 52 vorhandener Positionierwulst 48 den Ringwulst passieren, was zwar dem Zurückziehen kurzzeitig einen erhöhten Widerstand entgegensetzte, aber schließlich durch die in Fig. 13 dargestellte Form des Positionierwulstes 48 und durch das radial nach innen federnde Nachgeben der Halteklauen ermöglicht wurde.

In der in Fig. 12 dargestellten hinteren Endlage stehen die Anschlagnocken 16 am inneren Ringwulst 5 an, wie dies aus der vergrößerten Darstellung gemäß Fig. 13 deutlich ersichtlich ist. Nach der Trennung der Kolbenstange 11 verhindert der Positionierwulst 48 im Zusammenwirken mit dem inneren Ringwulst 5, dass die Nadelbaugruppe 25 mit der Kupplungshülse 51 in den Spritzenzylinder zurück geschoben wird. Auch bei diesem Beispiel kann der getrennte Teil der Kolbenstange 11 mittels der Endhülse 22 auf den Hals 8 des Spritzenzylinders 1 gesetzt werden, um diesen abzuschließen und Verletzungen durch die Injektionsnadel 27 sowie ein Heraustropfen von Resten der Injektionslösung 50 zu verhindern.

Die Ausführungsbeispiele zeigen mögliche Ausführungsvarianten der Injektionsspritze, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

Der Ordnung halber sei abschließend darauf hingewiesen, dass zum besseren Verständnis des Aufbaus der Injektionsspritze diese bzw. deren Bestandteile teilweise unmaßstäblich und/oder vergrößert und/oder verkleinert dargestellt wurden.

Die den eigenständigen erfinderischen Lösungen zugrunde liegende Aufgabe kann der Beschreibung entnommen werden.

Vor allem können die einzelnen in den Fig. 1 bis 9; 10; 11; 12, 13 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

### Bezugszeichenaufstellung

- 1: Spritzenzylinder
- 2: Flansch
- 3: Arretierungswulst
- 4: Schulter
- 5: Ringwulst

- 6: vordere Flanke
- 7: hintere Flanke
- 8: Hals
- 9: Nadelschutzkappe
- 10: Kolbenbaugruppe

- 11: Kolbenstange
- 12: Kolbenstopfen
- 13: Rastnocken
- 14: Ausnehmung
- 15: Federsteg

- 16: Anschlagnocken
- 17: vordere Platte
- 18: hintere Platte
- 19: Schaft
- 20: Spitze

- 21: Ansatz
- 22: Endhülse
- 23: Sollbruchstelle
- 24: Membrane
- 25: Nadelbaugruppe

- 26: Nadelhalter
- 27: Injektionsnadel
- 28: Kopf
- 29: Längsrippen
- 30: Ringnut

- 31: Bund
- 32: Schlitz
- 33: Ringnut
- 34: Ausnehmung
- 35 36: Dichteinsatz
- 37: Fortsatz
- 38: Schwächung
- 39: Hohlraum
- 40: Ringbund

- 41: Öffnung
- 42: Ringwulst
- 43: Dichtwulst
- 44: Absatz
- 45: Kragen

- 46: Aufnahmezylinder
- 47: Ringnut
- 48: Positionierwulst
- 49: Einschnürung
- 50: Injektionslösung

- 51: Kupplungshülse
- 52: Halteklauen
- 53: Einschnitte
- 54: Kupplungskopf
- 55: Hinterschnitt

## Patentansprüche

1. Injektionsspritze für den Einmalgebrauch, mit einem Spritzenzylinder (1), einem durch eine Kolbenstange (11) im Spritzenzylinder (1) verschiebbaren Kolbenstopfen (12), einer im Spritzenzylinder (1) aufgenommene Nadelbaugruppe (25), wobei diese einen eine Injektionsnadel (27) aus nicht rostendem Stahl auf einem Teil ihrer Länge umschließenden Nadelhalter (26) aus Kunststoff sowie einen Dichteinsatz (36) umfasst, und mit Kupplungsmitteln (20, 40) zum Verbinden der Kolbenstange (11) mit der Nadelbaugruppe (25), um nach erfolgter Injektion die Nadelbaugruppe (25) in den Spritzenzylinder (1) zurück zu ziehen, und der Spritzenzylinder (1), die Nadelbaugruppe (25) sowie der Kolbenstopfen (12) einen Innenraum zur Aufnahme einer Injektionslösung (50) bilden, **dadurch gekennzeichnet, dass** der Innenraum im Ausgangszustand mit der Injektionslösung (50) vorgefüllt ist, wobei auf ein dem Innenraum zugewendetes hinteres Ende des Nadelhalters (26) der aus Pharmagummi bestehende Dichteinsatz (36) aufgesetzt ist und dieser den Nadelhalter (26) gegen den die Injektionslösung (50) aufnehmenden Innenraum hin vollständig abdeckt und der Dichteinsatz (36) vom hinteren Ende der im Nadelhalter (26) gehaltenen Injektionsnadel (27) hin zum Innenraum durchragt ist und dass der Nadelhalter (26) an einem Bereich seines Außenmantels mit Längsrippen (29) ausgestattet ist und diese in einem verengten Endbereich (8) des Spritzenzylinders (1) aufgenommen sind sowie an diesem anliegen und dass an einem vorderen Ende eines Kragens (45) des Dichteinsatzes (36) ein Dichtwulst (43) angeordnet ist und der Dichtwulst (43) dichtend an der Innenwand des Spritzenzylinders (1) anliegt und diesen bakteriendicht verschließt.

2. Injektionsspritze nach Anspruch 1, **dadurch gekennzeichnet, dass** der Nadelhalter (26) an seinem injektionsseitigen Ende einen im Durchmesser erweiterten Kopf (28) aufweist, auf den eine Nadelschutzkappe (9) aufsetzbar ist.

3. Injektionsspritze nach Anspruch 1 oder 2, **dadurch gekennzeichnet, dass** der Nadelhalter (26) an seinem von der Injektionsseite abgewandten Ende eine stirnseitige Ausnehmung (34) hat, in welche das von der Injektionsseite abgewandte Ende der Injektionsnadel (27) ragt..

4. Injektionsspritze nach Anspruch 3, **dadurch gekennzeichnet, dass** in der die Ausnehmung (34) umgebenden Wandung des Nadelhalters (26) mindestens ein im Wesentlichen axial verlaufender Schlitz (32) angeordnet ist.

5. Injektionsspritze nach einem der Ansprüche 3 oder 4, **dadurch gekennzeichnet, dass** der Dichteinsatz (36) sich mit einem Fortsatz (37) in die Ausnehmung (34) des Nadelhalters (26) erstreckt und den Außenmantel des Nadelhalters (26) im Bereich der Ausnehmung (34) mit einem Kragen (45) umschließt.

6. Injektionsspritze nach einem der Ansprüche 3 oder 5, **dadurch gekennzeichnet, dass** der Dichteinsatz (36) im von der Injektionsseite abgewandten Endbereich seines Fortsatzes (37) einen Ringwulst (42) hat, der in eine in der Innenwand der Ausnehmung (34) vorgesehene Ringnut (33) greift.

7. Injektionsspritze nach einem der Ansprüche 5 oder 6, **dadurch gekennzeichnet, dass** der am Kragen (45) des Dichteinsatzes (36) angeordnete Dichtwulst (43) O-Ring ähnlich ausgebildet ist und in eine am Nadelhalter (26) angeordnete Ringnut (30) eingreift.

8. Injektionsspritze nach einem der vorangehenden Ansprüche, **dadurch gekennzeichnet, dass** am Außenumfang des Dichteinsatzes (36) ein Ansatz (44) gebildet ist, mit dem der Dichteinsatz (36) an einer im injektionsseitigen Bereich des Spritzenzylinders (1) gebildeten Schulter (4) axial anliegt.

9. Injektionsspritze nach einem der vorangehenden Ansprüche, **dadurch gekennzeichnet, dass** im Dichteinsatz (36) ein Hohlraum (39) vorhanden ist, von welchem aus sich eine Öffnung (41) unter Bildung eines Ringbunds (40) zum Innenraum des Spritzenzylinders (1) erstreckt, wobei sich das hintere Ende der Injektionsnadel (27) in diesen Hohlraum (39) erstreckt.

10. Injektionsspritze nach einem der vorangehenden Ansprüche, **dadurch gekennzeichnet, dass** nahe bei dem von der Injektionsseite abgewandten Ende des Spritzenzylinders ein innerer Ringwulst (5) angeordnet ist, dessen vordere, der Injektionsseite zugewandte Flanke (6) mindestens annähernd rechtwinklig zur Wand des Spritzenzylinders (1) ausgerichtet ist und dessen hintere, der Injektionsseite abgewandte Flanke (7) mit der Wand des Spritzenzylinders (1) einen stumpfen Winkel einschließt.

11. Injektionsspritze nach Anspruch 10, **dadurch gekennzeichnet, dass** an der Kolbenstange (11) Anschlagnocken (16) vorhanden sind, die eine von der Injektionsseite abgewandte, im Wesentlichen rechtwinklig zur Längsachse der Kolbenstange ausgerichtete Flanke aufweisen, die beim Zurückziehen der Kolbenstange (11) an der vorderen, der Injektionsseite zugewandten Flanke (6) des inneren Ringwulstes (5) anstehen und dadurch verhindern, dass die Kolbenstange ganz aus dem Spritzenzylinder (1) herausgezogen werden kann.

12. Injektionsspritze nach Anspruch 11, **dadurch gekennzeichnet, dass** an der Kolbenstange (11) in einem Abstand von den Anschlagnocken (16) auf der von der Injektionsseite abgewandten Seite Rastnocken (13) angeordnet sind, deren Flanken so ausgebildet sind, dass die Rastnocken (13) unter Überwindung eines Widerstands in beiden Richtungen am inneren Ringwulst (5) des Spritzenzylinders vorbei bewegt werden können.

13. Injektionsspritze nach Anspruch 12, **dadurch gekennzeichnet, dass** die Kolbenstange (11) im Bereich der Rastnocken (13) Ausnehmungen (14) aufweist, damit die Rastnocken (13) radial federnd nachgeben können.

14. Injektionsspritze nach einem der Anspruche 11 bis 13, **dadurch gekennzeichnet, dass** an der Kolbenstange (11) in einem Bereich zwischen den Anschlagnocken (16) und den Rastnocken (13) eine Sollbruchstelle (23) vorhanden ist.

15. Injektionsspritze nach einem der Ansprüche 1 bis 11, **dadurch gekennzeichnet, dass** die Kolbenstange (11) aus zwei Teilen besteht, die durch weitere Kupplungsmittel (51, 54) lösbar miteinander verbunden sind, wobei die Kupplungsmittel derart ausgebildet sind, dass sie ausschließlich in einer Betriebslage lösbar sind, in der die Kolbenstange bis zu einem Anschlag (5, 16) zurück gezogen ist.

16. Injektionsspritze nach Anspruch 15, **dadurch gekennzeichnet, dass** die Kupplungsmittel eine mit dem Kolbenstopfen (12) verbundene Kupplungshülse (51) enthalten, an der von der Injektionsseite abgewandte Kupplungsklauen (52) angeformt sind, welche einen Kupplungskopf (54) hintergreifen, wobei der Kupplungskopf (54) nur dann durch die radial federnd nachgiebigen Halteklauen (52) freigebbar ist, wenn diese sich auf mindestens einem Teil ihrer Länge außerhalb des Spritzenzylinders (1) oder in einem Endbereich des Spritzenzylinders
mit vergrößertem Durchmesser befinden.

17. Injektionsspritze nach einem der vorangehenden Ansprüche, **dadurch gekennzeichnet, dass** am von der Injektionsseite abgewandten hinteren Ende der Kolbenstange eine Endhülse (22) angeordnet ist, deren nach hinten gerichtete Öffnung einen auf das vordere Ende des Spritzenzylinders passenden Durchmesser aufweist.

## Claims

1. Syringe for one-off use, with a syringe barrel (1), a barrel stopper (12) which can be displaced in the syringe barrel (1) by a plunger rod (11), a needle unit (25) accommodated in the syringe barrel (1), the latter comprising a needle holder (26) made from plastic surrounding an injection needle (27) made from stainless steel across a part of its length as well as a seal insert (36), and with coupling means (20, 40) for connecting the plunger rod (11) to the needle unit (25) in order to retract the needle unit (25) into the syringe barrel (1) after the injection, the syringe barrel (1), needle unit (25) and plunger stopper (22) forming an interior for accommodating an injection solution (50), **characterised in that** in the initial state, the interior is pre-filled with the injection solution (50), and the seal insert (36) made from pharmaceutical rubber is fitted on a rear end of the needle holder (26) facing the interior and completely separates the needle holder (26) from the interior accommodating the injection solution (50), and the injection needle (27) retained in the needle holder (26) extends through the seal insert (36) from the rear end as far as the interior and the needle holder (26) is provided with longitudinal ribs (29) in a region of its external face which are accommodated in a narrower end region (8) of the syringe barrel (1) and lie against the latter, and a sealing bead (43) is disposed on a front end of a collar (45) of the seal insert (36), and the sealing bead (43) lies in a sealing arrangement against the internal wall of the syringe barrel (1), thereby sealing it off from bacteria.

2. Syringe as claimed in claim 1, **characterised in that** the needle holder (26) has a head (28) of a wider diameter at its injection end on which a needle guard cap (9) can be fitted.

3. Syringe as claimed in claim 1 or 2, **characterised in that** the needle holder (26) has a cut-out (34) in its end face remote from the injection end, through which the end of the injection needle (27) remote from the injection end extends.

4. Syringe as claimed in claim 3, **characterised in that** at least one essentially axially extending slot (32) is provided in the wall of the needle holder (26) surrounding the cut-out (34).

5. Syringe as claimed in one of claims 3 or 4, **characterised in that** the seal insert (36) projects into the cut-out (34) of the needle holder (26) by means of an extension (37) and surrounds the external face of the needle holder (26) in the region of the cut-out (34) by means of a collar (45).

6. Syringe as claimed in one of claims 3 or 5, **characterised in that** the seal insert (36) has an annular bead (42) in the end region of its extension (37) remote from the injection end, which locates in an annular groove (33) provided in the internal wall of the cut-out (34).

7. Syringe as claimed in one of claims 5 or 6, **characterised in that** the sealing bead (43) disposed on the collar (45) of the seal insert (36) is of a design similar to an O-ring and locates in an annular groove (30) disposed in the needle holder (26).

8. Syringe as claimed in one of the preceding claims, **characterised in that** a shoulder (44) is formed on the external circumference of the seal insert (36), by means of which the seal insert (36) sits axially against a shoulder (4) disposed in the injection-end region of the syringe barrel (1).

9. Syringe as claimed in one of the preceding claims, **characterised in that** a cavity (39) is provided in the seal insert (36), from which an orifice (41) extends as far as the interior of the syringe barrel (1) forming an annular collar (40), and the rear end of the injection needle (27) extends through this cavity (39).

10. Syringe as claimed in one of the preceding claims, **characterised in that** an inner annular bead (5) is disposed close to the end of the syringe barrel remote from the injection end, the front face (6) of which directed towards the injection end is oriented at least approximately at a right angle with respect to the wall of the syringe barrel (1) and the rear face (7) of which remote from the injection end subtends an obtuse angle with the wall of the syringe barrel (1).

11. Syringe as claimed in claim 10, **characterised in that** stop cams (16) are provided on the plunger rod (11), which have a face remote from the injection end oriented essentially at a right angle to the longitudinal axis of the plunger rod, which sit against the front face (6) of the inner annular bead (5) directed towards the injection end when the plunger rod (11) is retracted and thus make it impossible for the plunger rod to pulled completely out of the syringe barrel (1).

12. Syringe as claimed in claim 11, **characterised in that** catch cams (13) are provided on the plunger rod (11) at a distance from the stop cams (16) at the end remote from the injection end, the faces of which are designed so that the catch cams (13) can be moved past the inner annular bead (5) of the syringe barrel by overcoming a resistance in both directions.

13. Syringe as claimed in claim 12, **characterised in that** the plunger rod (11) has cut-outs (14) in the region of the catch cams (13), thereby enabling the catch cams (13) to flex radially.

14. Syringe as claimed in one of claims 11 to 13, **characterised in that** a breaking point (23) is provided on the plunger rod (11) in a region between the stop cams (16) and the catch cams (13).

15. Syringe as claimed in one of claims 1 to 11, **characterised in that** the plunger rod (11) comprises two parts which are releasably connected to one another by other coupling means (51, 54) and the coupling means are designed so that they can be released exclusively in an operating position in which the plunger rod has been retracted as far as a stop (5, 16).

16. Syringe as claimed in claim 15, **characterised in that** the coupling means contain a coupling sleeve (51) connected to the plunger stopper (12) on which coupling claws (52) are formed remote from the injection end, which locate behind a coupling head (54), and the coupling head (54) can only be released by the radially flexible retaining claws (52) when the latter are disposed with at least a part of their length outside the syringe barrel (1) or in an end region of the syringe barrel with a larger diameter.

17. Syringe as claimed in one of the preceding claims, **characterised in that** a terminal sleeve (22) is disposed on the end of the plunger rod remote from the injection end, the rearwardly oriented orifice of which has a diameter matching the front end of the syringe barrel.

## Revendications

1. Seringue d'injection à usage unique, avec un cylindre de seringue (1), un bouchon de piston (12) déplaçable dans le cylindre de seringue (1) par une tige de piston (11), un module d'aiguille (25) reçu dans le cylindre de seringue (1), où celui-ci comprend un porte-aiguille (26) en matériau synthétique renfermant une aiguille d'injection (27) en acier inoxydable sur une partie de sa longueur ainsi qu'un insert d'étanchéité (36), et avec des moyens de couplage (20, 40) pour relier la tige de piston (11) au module d'aiguille (25) pour, après l'injection, retirer le module d'aiguille (25) dans le cylindre de seringue (1), et le cylindre de seringue (1), le module d'aiguille (25) et le bouchon de piston (12) forment un espace intérieur pour la réception d'une solution d'injection (50), **caractérisée en ce que** l'espace intérieur, à l'état de départ, est prérempli avec la solution d'injection (50), où est placé sur une extrémité arrière du porte-aiguille (26), orientée vers l'espace intérieur, l'insert d'étanchéité (36) réalisé en caoutchouc pharmaceutique, et celui-ci recouvre complètement le porte-aiguille (26) relativement à l'espace intérieur recevant la solution d'injection (50), et l'insert d'étanchéité (36) est traversé par l'extrémité arrière de l'aiguille d'injection (27) retenue dans le porte-aiguille (26) vers l'espace intérieur, et **en ce que** le porte-aiguille (26) présente à une zone de son enveloppe extérieure des nervures longitudinales (29), et celles-ci sont reçues dans une zone d'extrémité retrécie (8) du cylindre de seringue (1) et s'appliquent à celui-ci, et **en ce qu'**il est disposé à une extrémité avant d'un collet (45) de l'insert d'étanchéité (36) un bourrelet d'étanchéité (43), et le bourrelet d'étanchéité (43) s'applique d'une manière étanche à la paroi intérieure du cylindre de seringue (1) et ferme celui-ci d'une manière étanche aux bactéries.

2. Seringue d'injection selon la revendication 1, **caractérisée en ce que** le porte-aiguille (26) présente à son extrémité côté injection une tête (28) d'un plus grand diamètre sur laquelle peut être placé un capuchon de protection d'aiguille (9).

3. Seringue d'injection selon la revendication 1 ou 2, **caractérisée en ce que** le porte-aiguille (26) présente à son extrémité éloignée du côté d'injection un évidement frontal (34) dans lequel fait saillie l'extrémité de l'aiguille d'injection (27) éloignée du côté d'injection.

4. Seringue d'injection selon la revendication 3, **caractérisée en ce qu'**est ménagée dans la paroi du porte-aiguille (26) entourant l'évidement (34) au moins une fente (32) s'étendant sensiblement axialement.

5. Aiguille d'injection selon l'une des revendications 3 ou 4, **caractérisée en ce que** l'insert d'étanchéité (36) s'étend avec un prolongement (37) dans l'évidement (34) du porte-aiguille (26) et entoure l'enveloppe extérieure du porte-aiguille (26) dans la zone de l'évidement (34) avec un collet (45).

6. Seringue d'injection selon l'une des revendications 3 ou 5, **caractérisée en ce que** l'insert d'étanchéité (36) présente dans la zone d'extrémité, éloignée du côté d'injection, de son prolongement (37) un bourrelet annulaire (42) qui s'engage dans une rainure annulaire (33) prévue dans la paroi intérieure de l'évidement (34).

7. Aiguille d'injection selon l'une des revendications 5 ou 6, **caractérisée en ce que** le bourrelet d'étanchéité (43) disposé au collet (45) de l'insert d'étanchéité (36) est réalisé en forme de joint torique et s'engage dans une rainure annulaire (30) disposée au porte-aiguille (26).

8. Seringue d'injection selon l'une des revendications précédentes, **caractérisée en ce qu'**est formé sur le pourtour extérieur de l'insert d'étanchéité (36) un bout rapporté (44) par lequel l'insert d'étanchéité (36) s'applique axialement à un épaulement (4) formé dans la zone côté injection du cylindre de seringue (1).

9. Cylindre d'injection selon l'une des revendications précédentes, **caractérisé en ce qu'**il existe dans l'insert d'étanchéité (36) un espace creux (39) à partir duquel s'étend une ouverture (41) en formant un collet annulaire (40) vers l'espace intérieur du cylindre de seringue (1), où l'extrémité arrière de l'aiguille d'injection (27) s'étend dans cet espace creux (39).

10. Aiguille d'injection selon l'une des revendications précédentes, **caractérisée en ce qu'**il est disposé à proximité de l'extrémité du cylindre de seringue éloignée du côté d'injection un bourrelet annulaire intérieur (5), dont le flanc avant (6) orienté vers le côté d'injection est orienté au moins approximativement perpendiculairement à la paroi du cylindre de seringue (1), et dont le flanc arrière (7), éloigné du côté d'injection, forme avec la paroi du cylindre de seringue (1) un angle obtus.

11. Seringue d'injection selon la revendication 10, **caractérisée en ce que** sont prévus à la tige de piston (11) des ergots de butée (16) qui présentent un flanc éloigné du côté d'injection, orienté sensiblement perpendiculairement à l'axe longitudinal de la tige de piston qui, lors du retrait de la tige de piston (11), s'appliquent au flanc avant (6) orienté vers le côté d'injection du bourrelet annulaire intérieur (5) et empêchent ainsi que la tige de piston puisse être sortie complètement du cylindre de seringue (1).

12. Seringue d'injection selon la revendication 11, **caractérisée en ce que** sont disposés à la tige de piston (11), à une distance des ergots de butée (16), au côté éloigné du côté d'injection, des ergots d'enclenchement (13) dont les flancs sont réalisés de telle sorte que les ergots d'enclenchement (16), en surmontant une résistance, peuvent être amenés à passer dans les deux directions devant le bourrelet annulaire intérieur (5) du cylindre de seringue.

13. Seringue d'injection selon la revendication 12, **caractérisée en ce que** la tige de piston (11) présente dans la zone des ergots d'enclenchement (13) des évidements (14) pour que les ergots d'enclenchement (13) puissent céder radialement élastiquement.

14. Seringue d'injection selon l'une des revendications 11 à 13, **caractérisée en ce qu'**est prévu à la tige de piston (11) dans une zone entre les ergots de butée (16) et les ergots d'enclenchement (13) un emplacement de rupture de consigne (23).

15. Seringue d'injection selon l'une des revendications 1 à 11, **caractérisée en ce que** la tige de piston (11) est constituée de deux parties par lesquelles d'autres moyens de couplage (51, 54) sont reliés amoviblement entre eux, où les moyens de couplage sont réalisés de telle sorte qu'ils peuvent être relâchés exclusivement dans une position de fonctionnement dans laquelle la tige de piston a été retirée jusqu'à une butée (5, 16).

16. Seringue d'injection selon la revendication 15, **caractérisée en ce que** les moyens de couplage comprennent une douille de couplage (51) reliée au bouchon de piston (12), à laquelle sont rapportées par formage des griffes de couplage (52) éloignées du côté d'injection, qui passent derrière une tête de couplage (54), où la tête de couplage (54) peut être libérée alors seulement par les griffes de retenue (52) cédant radialement élastiquement lorsque celles-ci se trouvent sur au moins une partie de leur longueur à l'extérieur du cylindre de seringue (1) ou dans une zone d'extrémité du cylindre de seringue d'un diamètre agrandi.

17. Seringue d'injection selon l'une des revendications précédentes, **caractérisée en ce qu'**est disposée à l'extrémité arrière de la tige de piston, éloignée du côté d'injection, une douille d'extrémité (22) dont l'ouverture dirigée vers l'arrière présente un diamètre adapté à l'extrémité avant du cylindre de seringue.
